# EUROPÄISCHE PATENTSCHRIFT

(11) **EP 3 373 793 B1**
(45) Veröffentlichungstag und Bekanntmachung des Hinweises auf die Patenterteilung: **08.12.2021**
(21) Anmeldenummer: 16787827.1
(22) Anmeldetag: 26.10.2016
(51) Int. Cl.: A61B 1/00, A61B 1/04, A61B 46/10

(54) **STERILADAPTER**
STERILE ADAPTER
ADAPTATEUR STÉRILE

(30) Priorität: 13.11.2015 DE 102015119695
(43) Veröffentlichungstag der Anmeldung: 19.09.2018
(73) Patentinhaber: XION GmbH, 13127 Berlin (DE)
(72) Erfinder: LASER, Helmut, 13156 Berlin (DE); MÜLLER, Holger, 16548 Glienicke-Nordbahn (DE)
(74) Vertreter: Eisenführ Speiser
(86) Internationale Anmeldenummer: PCT/EP2016/075743
(87) Internationale Veröffentlichungsnummer: WO 2017/080821

(56) Entgegenhaltungen:
- EP-A1- 0 940 116
- EP-A1- 2 759 285
- CN-U- 201 976 163
- GB-A- 2 495 561
- US-A1- 2014 051 923

## Beschreibung

Die Erfindung betrifft einen Steriladapter zur Anordnung zwischen einem sterilisierbaren Endoskop und einem Kamerakopf.

Bei der medizinischen Endoskopie müssen durch den Patienten oder Operateur berührbare Instrumente steril sein. Die verbreitete Dampfsterilisation stellt jedoch einen erheblichen Aufwand dar. Eine häufig verwendete Anordnung in der Endoskopie ist eine Kombination aus einem Endoskop und einem daran anschließbaren Kamerakopf. Bei dieser Anordnung wird üblicherweise das Endoskop sterilisiert, der Kamerakopf jedoch mit einem Sterilüberzug zur eimaligen Verwendung abgedeckt. Hierbei kann auch ein Zwischenadapter zwischen Endoskop und Kamerakopf angeordnet werden. Die bekannten Anordnungen nutzen ein transparentes Fenster oder eine transparente Schutzfolie zur Trennung von steriler und unsteriler Seite.

Aus US 5,498,230A und EP 0 955 014 A1 sind Steriladapter mit transparentem Fenster bekannt. Aus US 2014/051923 und GB2495561 sind Steriladapter zwischen einem Endoskop und einer tragbaren Telefonkamera bekannt.

Aus US 5,882,295 A und US 5,433,221 A sind transparente Sterilüberzüge bekannt, die zwischen Endoskop und Kamerakopf mit einer transparenten Folie eine Sterilbarriere herstellen.

Aufgabe der Erfindung ist es, einen verbesserten Steriladapter bereitzustellen.

Erfindungsgemäß wird hierzu ein Steriladapter zur Anordnung zwischen einem sterilisierbaren Endoskop und einem Kamerakopf vorgeschlagen, mit einem an das Endoskop anschließbaren distalen Ende und einem an den Kamerakopf anschließbaren proximalen Ende. Dabei weist der Steriladapter einen Tubus mit einer Längsachse auf, bei dem ein Längsabschnitt des Tubus einen in einer Querschnittebene senkrecht zur Längsachse verringerten Innendurchmesser aufweist, der eine zentrale Öffnung bildet, die so bemessen ist, dass eine freie Lichtausbreitung zwischen dem Endoskop und dem Kamerakopf entlang der Längsachse möglich aber ein Durchgreifen durch einen von dem Tubus eingeschlossenen Innenraum des Steriladapters erschwert ist, wobei die zentrale Öffnung des Längsabschnitts einen Innendurchmesser von höchstens 10mm aufweist, und wobei der Tubus an seinem distalen Ende einen Innendurchmesser von weniger als 30mm hat und ein Abstand der Öffnung von dem distalen Ende des Tubus größer ist als die Hälfte eines Innendurchmessers des Tubus an dessen distalem Ende und der Tubus sich vom distalen Ende zum proximalen Ende erstreckt und der Längsabschnitt des Tubus das proximale Ende bildet.

Ein Vorteil der Erfindung liegt darin, dass der Steriladapter eine freie Lichtausbreitung zwischen Endoskop und Kamerakopf erlaubt, so dass eine durch den Kamerakopf aufgenommene Bildqualität des Endoskops nicht durch den Adapter verringert wird. Insbesondere sorgt die freie Lichtausbreitung durch die Öffnung des Steriladapters dafür, dass keine Verschmutzungen, Schlieren oder Doppelbrechung an optischen Komponenten des Steriladapters die Bildqualität beeinträchtigen, wie dies beispielsweise für aus dem Stand der Technik bekannte transparente Fenster oder transparente Schutzfolien möglich sein kann. Freie Lichtausbreitung bedeutet also im Folgenden, dass Licht sich nicht an festen Materialen, wie einer Folie oder einem Fenster brechen muss, sondern zumindest Teile eines sich zwischen Kamerakopf und Endoskop ausbreitenden Lichtstrahls ungehindert sind.

Dadurch, dass der erfindungsgemäße Steriladapter keine Flächen aufweist, durch die Licht geführt wird, entfällt eine Reinigung solch optischer Flächen im Rahmen einer Vorbereitung einer Nutzung des Steriladapters.

Zusätzlich wird ein Übertragen von Keimen von dem unsterilen Kamerakopf vermieden, da ein Kontakt mit dem Kamerakopf durch den Steriladapter erschwert wird. Insbesondere wird in einer sterilen Umgebung, beispielsweise im Rahmen einer medizinischen Operation, ein versehentlicher Kontakt mit dem unsterilen Kamerakopf vermieden. Dies kann auch vorteilhaft sein, um während einer Operation das Endoskop zu wechseln, ohne eine sterile Operationsumgebung den Keimen des Kamerakopfs auszusetzen.

Der Steriladapter kombiniert somit ein Ermöglichen von freier Lichtausbreitung mit einem Schutz gegenüber dem Kontakt einer sterilen Umgebung mit dem unsterilen Kamerakopf.

Dabei ist der Durchmesser der Öffnung vorteilhaft so gewählt, dass man nicht aus Versehen durch die Öffnung an den unsterilen Kamerakopf greift.

Dadurch dass der Abstand der Öffnung von dem distalen Ende des Tubus größer ist als die Hälfte eines Innendurchmessers des Tubus an dessen distalem Ende, wird ein Öffnungswinkel des Steriladapters beschrieben, der zwischen dem distalen Ende und der zentralen Öffnung besteht, und der ausgehend von einem Mittelpunkt der Öffnung höchstens 90° beträgt.

Insbesondere für nicht kreisförmige Querschnitte bedeutet der Begriff Durchmesser oder Innendurchmesser im Folgenden eine minimale Ausdehnung in einer Richtung senkrecht zur Längsachse. Die Begriffe Durchmesser und Innendurchmesser sollen allerdings nicht implizieren, dass die Öffnung oder der Tubus kreisförmig sind. Während die Öffnung in einer bevorzugten Ausführungsform kreisförmig ist, ist die Öffnung in anderen Ausführungsformen eckig oder rund. Insbesondere kann die Öffnung auch rechteckig, ellipsoid oder sternförmig sein.

Nachfolgend werden bevorzugte Ausführungsvarianten des Steriladapters vorgeschlagen.

In einer besonders bevorzugten Ausführungsvariante weist die zentrale Öffnung des Längsabschnitts einen Innendurchmesser zwischen 1mm und 10mm, insbesondere zwischen 4mm und 8mm auf. Da typische Strahlenbreiten des Endoskops im Bereich von einem Millimeter liegen, ist diese Ausführungsvariante besonders vorteilhaft, um sowohl eine freie Lichtausbreitung zwischen Endoskop und Kamerakopf zu ermöglichen als auch einen Durchgriff, beispielsweise durch Finger eines Nutzers des Endoskops, zu vermeiden. Ein kleinerer Innendurchmesser reduziert die Möglichkeit, mit dem Kamerakopf in Kontakt zu gelangen. Allerdings können für stereoendoskopische Anwendungen Innendurchmesser zwischen 4mm und 8mm besser geeignet sein als Innendurchmesser zwischen 1mm und 3mm, da zwei Strahlengänge durch den Kamerakopf abzubilden sind.

Vorzugsweise weist der Tubus in Richtung der Längsachse eine Ausdehnung von mindestens 10mm und insbesondere mehr als 30mm auf. Dies vergrößert eine Erstreckung des Adapters und führt daher zu einer Vermeidung eines Durchgreifens des von dem Tubus umgebenen Volumens des Steriladapters. Eine Ausdehnung des Tubus entsprechend dieser Ausführungsvariante kann dazu beitragen, dass trotz eines versehentlichen Kontakts mit der Innenkontur des Steriladapters kein Kontakt mit dem unsterilen Kamerakopf hergestellt wird.

Vorzugsweise weist der Längsabschnitt mit verringertem Innendurchmesser in Richtung der Längsachse eine Breite zwischen 0,5mm und 2mm auf.

In einer besonders bevorzugten Ausführungsvariante ist der Längsabschnitt mit verringertem Innendurchmesser als Lochblende ausgebildet, d.h. die Ausdehnung des Längsabschnitts mit verringertem Innendurchmesser in Längsrichtung ist kleiner als der Durchmesser der zentralen Öffnung. Der Steriladapter dieser Ausführungsform kann besonders einfach hergestellt werden, beispielsweise durch ein Fließpressverfahren.

Vorzugsweise ist der erfindungsgemäße Steriladapter aus Edelstahl gefertigt. In anderen Varianten ist der Steriladapter aus Aluminium, Hartplastik oder einem anderen Kunststoff gefertigt.

Für eine Herstellung des Steriladapters ist es besonders vorteilhaft, wenn dieser monolithisch hergestellt ist. Hierbei muss kein zusätzliches Befestigungsmittel zum Befestigen des Längsabschnitts oder zum Befestigen einer Lochblende mit dem Tubus vorgesehen werden.

In einer besonders bevorzugten Ausführungsvariante ist der Steriladapter weitgehend rotationssymmetrisch zur Längsachse ausgebildet. Hierbei ist eine besonders einfache Herstellung möglich. In dieser Ausführungsvariante ist die Verjüngung der Innenkontur vorzugsweise als Lochblende ausgebildet. Abweichungen von einer rotationssymmetrischen Ausbildung können beispielsweise durch eine Verbindung für ein Verbinden mit dem sterilisierbaren Endoskop oder dem Kamerakopf vorgesehen sein. Ein Steriladapter dieser Ausführungsvariante kann beispielsweise eine äußere Mantelfläche des Tubus aufweisen, die in Längsrichtung parallel bezüglich der Längsachse verläuft oder die konisch ausgebildet ist.

In einer Ausführungsvariante hat der Tubus in Richtung der Längsachse eine Ausdehnung, die größer als das 0,8 fache des maximalen Innendurchmessers des Tubus in einer Richtung senkrecht zur Längsachse ist. Durch dieses Verhältnis aus Ausdehnung des Tubus und maximalem Innendurchmesser kann in dieser Ausführungsvariante erreicht werden, dass der Öffnungswinkel kleiner als 70° ist. Hierdurch kann ein versehentliches Durchgreifen eines von dem Tubus eingeschlossenen Innenraums des Steriladapters vermieden werden.

In einer besonders bevorzugten Ausführungsvariante ist die Öffnung an dem proximalen Ende des Steriladapters ausgebildet. Während der Kontakt mit dem Kamerakopf in dieser Ausführungsvariante weiterhin vermieden wird, sorgt ein in einer Nähe des Kamerakopfes an dem proximalen Ende des Steriladapters ausgebildeter verringerter Innendurchmesser entlang des Längsabschnitts, im Gegensatz zu einer Verjüngung am distalen Ende des Steriladapters für eine größere Apertur des an den Steriladapter angeschlossenen Kamerakopfes. Dies kann sich vorteilhaft auf die Bildqualität von durch den Kamerakopf aufgenommenen Daten des Endeskops auswirken.

In einer weiteren Ausführungsvariante ist an dem Tubus im Längsabschnitt ein beweglicher Verschluss der Öffnung angeordnet, der ausgebildet ist eine offene Verschlussstellung anzunehmen, wenn der Steriladapter mechanisch mit dem Endoskop verbunden ist, und eine geschlossene Verschlussstellung anzunehmen, wenn der Steriladapter nicht mechanisch mit dem Endoskop verbunden ist. Hierbei behindert die geschlossene Verschlussstellung die freie Lichtausbreitung zwischen dem distalen Ende und dem proximalen Ende des Steriladapters, während die offene Verschlussstellung die freie Lichtausbreitung erlaubt. In dieser Ausführungsvariante ist die sterile Umgebung besonders gut gegen Keime des unsterilen Kamerakopfes geschützt. So wird bei einem Wechsel des Endoskops die Gefahr eines Kontakts mit dem Kamerakopf sowohl durch den Tubus und den verringerten Innendurchmesser im Bereich des Längsabschnitts reduziert als auch durch den beweglichen Verschluss. Der bewegliche Verschluss kann einen versehentlichen Kontakt mit dem unsterilen Kamerakopf weitgehend ausschließen, da ein Kontakt dieses Kamerakopfes bei angeschlossenem Endoskop und somit offenem Verschluss für gewöhnlich nicht möglich ist. Zusätzlich weist der Steriladapter dieser Ausführungsvariante den Vorteil einer ungehinderten Lichtausbreitung zwischen Endoskop und Kamerakopf auf.

Mechanisch realisiert ist der bewegliche Verschluss in einer Ausführungsvariante über eine Feder, die mit einer an einer Innenkontur des Längsabschnitts gelagerten Klappe derart verbunden ist, dass bei einem Anlegen des Endoskops eine Federkraft der Feder die Klappe aufklappt, und wobei beim Lösen des Endoskops von dem Steriladapter die Federkraft der Feder verringert wird, was ein Zuklappen der Klappe bewirkt. In einer weiteren Ausführungsvariante wird der bewegliche Verschluss durch mindestens ein ebenes Blendenelement gebildet, wobei das Anlegen des Endoskops an den Steriladapter eine Verschiebung des Blendenelements in einer Ebene senkrecht zur Längsachse bewirkt, wodurch eine freie Lichtausbreitung zwischen Endoskop und Kamerakopf ermöglicht wird. Das Lösen des Endoskops sorgt hingegen in dieser Ausführungsvariante für ein Verschieben des Blendenelements in der Ebene senkrecht zur Längsachse, so dass der Verschluss in die geschlossene Verschlussstellung überführt ist. Das Blendenelement ist dabei bevorzugt nach Art einer Lamellenblende mit einer Anzahl von weiteren Blendenelementen an dem Steriladapter ausgebildet.

In einer bevorzugten Ausführungsvariante sind der Steriladapter und das Endoskop und/oder der Kamerakopf über eine durch den Tubus jeweils gebildete Steckverbindung aneinander anschließbar. Diese Steckverbindung kann dabei über eine zusätzliche Sicherung, beispielsweise durch einen lösbaren Rastmechanismus verfügen, um ein versehentliches Lösen des Endoskops oder des Kamerakopfes von dem Steriladapter zu verhindern.

In einer weiteren Ausführungsvariante sind der Steriladapter und das Endoskop und/oder der Kamerakopf über eine durch den Tubus jeweils gebildete Schraubverbindung aneinander anschließbar. Eine Schraubverbindung ermöglicht in vorteilhafter Weise eine sichere Verbindung zwischen Steriladapter und/oder Endoskop, wobei ein Risiko eines versehentlichen Lösens des Endoskops oder des Kamerakopfes besonders gering ist.

In einer weiteren Ausführungsvariante ist der Steriladapter mit dem Kamerakopf über eine Schraubverbindung befestigt und mit dem Endoskop über eine Steckverbindung. Dies kann einen einfachen und schnellen Wechsel des Endoskops in einer sterilen Umgebung über die Steckverbindung ermöglichen. Außerdem ist der Steriladapter mit dem Kamerakopf verschraubt, so dass ein versehentliches Lösen des Kamerakopfes von dem Steriladapter und ein resultierendes Verunreinigen einer sterilen Umgebung mit dem Kamerakopf vermieden werden kann.

Die Erfindung soll nun anhand von Ausführungsbeispielen mit Bezug auf die Figuren näher erläutert werden. Von den Figuren illustriert:
- Fig. 1: einen Querschnitt eines erfindungsgemäßen Ausführungsbeispiels des Steriladapters;
- Fig. 2: eine Vorderansicht des erfindungsgemäßen Ausführungsbeispiels des Steriladapters gemäß Fig. 1;
- Fig. 3: eine schematische Ansicht des Querschnitts aus Fig. 1 mit angeschlossenem Endoskop und Kamerakopf.

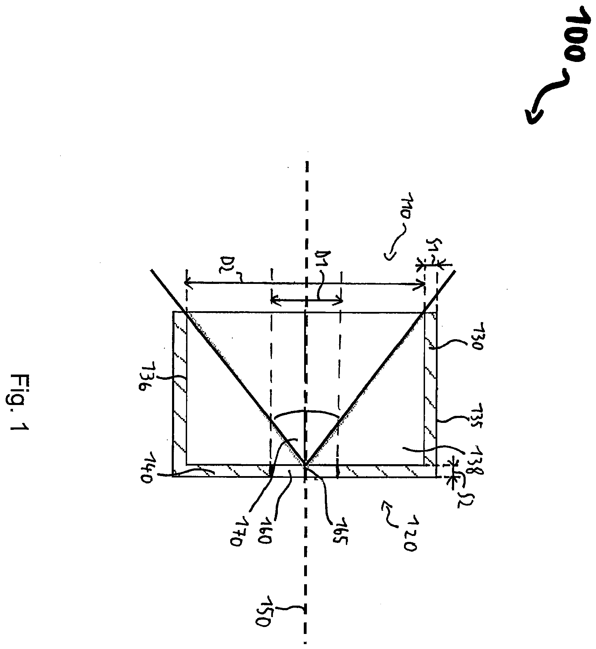

Fig. 1 zeigt einen Querschnitt eines erfindungsgemäßen Ausführungsbeispiels des Steriladapters 100.

Der Steriladapter 100 verfügt über ein distales Ende 110, an die ein Endoskop angeschlossen werden kann und ein proximales Ende 120, an die ein Kamerakopf angeschlossen werden kann. Dabei bildet ein Tubus 130 mit einer Längsachse 150 eine Mantelfläche 135 des Steriladapters 100 mit einer Innenkontur 136. Ein Längsabschnitt 140 weist einen in einer Querschnittsebene senkrecht zur Längsachse 150 verringerten Innendurchmesser D1 auf, der eine zentrale Öffnung 160 bildet. Die zentrale Öffnung 160 ist so bemessen, dass hierdurch eine freie Lichtausbreitung zwischen dem Endoskop an dem distalen Ende 110 und dem Kamerakopf an dem proximalen Ende 120 entlang der Längsachse 150 erlaubt wird, während ein Durchgreifen durch einen von dem Tubus 130 eingeschlossenen Innenraum 138 des Steriladapters 100 erschwert ist. Die zentrale Öffnung 160 des Längsabschnitts 140 weist einen Innendurchmesser D1 von höchstens 10mm auf, wobei der Tubus 130 an seinem distalen Ende 110 einen Innendurchmesser D2 von weniger als 30mm hat und ein Abstand der Öffnung 160 von dem distalen Ende 110 des Tubus 130 größer ist als die Hälfte eines Innendurchmessers D2 des Tubus 130 an dessen distalem Ende 110.

Der Tubus 130 beschreibt durch seine Ausdehnung hin zum distalen Ende 110 ausgehend von einem Mittelpunkt 165 der Öffnung 160 einen Öffnungswinkel 170 des Steriladapters 100. Dieser Öffnungswinkel 170 ist so ausgebildet, dass er höchstens 90° beträgt. Der Mittelpunkt 165 ist hierbei der geometrische Schwerpunkt einer durch die Öffnung 160 beschriebenen Form einer zum distalen Ende 110 des Steriladapters 100 gerichteten distalen Seite des Längsabschnitts 140.

In dem dargestellten Ausführungsbeispiel beträgt der Innendurchmesser D1 zwischen 4mm und 8mm. Weiterhin beträgt die Ausdehnung des Tubus 130 mindestens 10mm, bevorzugt 18mm.

Die durch den Tubus 130 gebildete Mantelfläche 135 ist axialsymetrisch zur Längsachse 150 und umgibt die Längsachse zylindermantelförmig, kann aber auch z.B. ganz oder abschnittsweise zylindermantelförmig oder auch ganz oder abschnittsweise konisch ausgebildet sein. Die Innenkontur 140 ist außerhalb eines Bereiches ihrer Verjüngung durch den verringerten Innendurchmesser D1 ebenfalls parallel zur Längsachse 150. Eine Wandstärke S1 des Tubus 130 zwischen Mantelfläche 135 und Innenkontur 140 liegt dabei zwischen 0,5mm und 2mm.

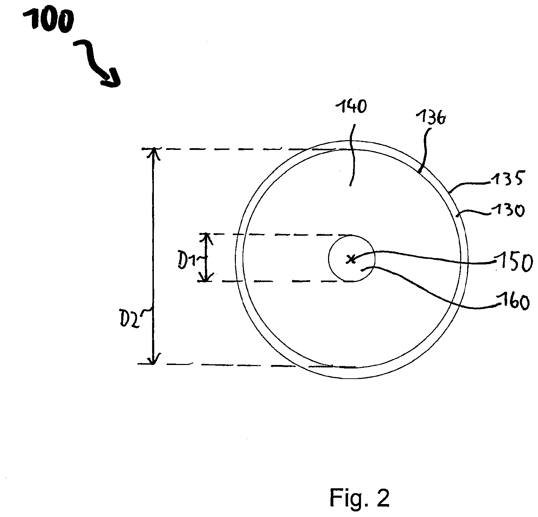

Der Längsabschnitt 140 ist durch den verringerten Innendurchmesser D1 als Lochblende mit der Öffnung 160 geformt. Die Lochblende ist dabei senkrecht zur Längsachse 150 ausgebildet. Derjenige Teil der Innenkontur 140, der innerhalb der Öffnung 160 liegt, ist parallel zur Mantelfläche 135 ausgebildet. Die durch den Bereich des verringerten Innendurchmessers D1 geformte Lochblende hat eine Stärke S2 zwischen 0,5mm und 2mm, wobei die Stärke S2 in dem gezeigten Ausführungsbeispiel identisch mit der Stärke S1 ist. Der Längsabschnitt 140 ist dabei an dem proximalen Ende 120 des Steriladapters 100 ausgebildet, derart, dass der Steriladapter 100 monolithisch ausgebildet und dabei rotationssymmetrisch zur Längsachse 150 ist, was jedoch nur aus Fig. 2 ersichtlich ist.

Die durch den Längsabschnitt 140 geformte Lochblende ist in dem dargestellten Ausführungsbeispiel an einem proximalen Rand des proximalen Endes 120 bündig an der umfänglichen Mantelfläche 135 ausgebildet. In einem nicht dargestellten Ausführungsbeispiel hat der Längsabschnitt 140 mit verringertem Innendurchmesser D1 einen identischen Abstand zwischen proximalem Ende und distalem Ende des Steriladapters.

Fig. 2 zeigt eine Vorderansicht des erfindungsgemäßen Ausführungsbeispiels des Steriladapters 100 gemäß Fig. 1.

Im Unterschied zu dem Querschnitt aus Fig. 1, verdeutlicht Fig. 2, dass der Steriladapter 100 rotationssymmetrisch zur Längsachse 150 ausgebildet ist. Dabei ist die Öffnung 160 kreisförmig ausgebildet und hat den Innendurchmesser D1. In anderen nicht dargestellten Ausführungsbeispielen hat die Öffnung eine polygonale oder ellipsoide Form, insbesondere eine Rechteck-Form.

Angesichts der Rotationssymmetrie ist der Tubus 130 kreisförmig ausgebildet. Er wird dabei begrenzt von der kreisförmig ausgebildeten Mantelfläche 135 und der ebenfalls kreisförmigen Innenkontur 136, die an dem distalen Ende den Innendurchmesser D2 hat.

Der Längsabschnitt 140 mit verringertem Innendurchmesser D1 ist als Lochblende mit der Öffnung 160 geformt.

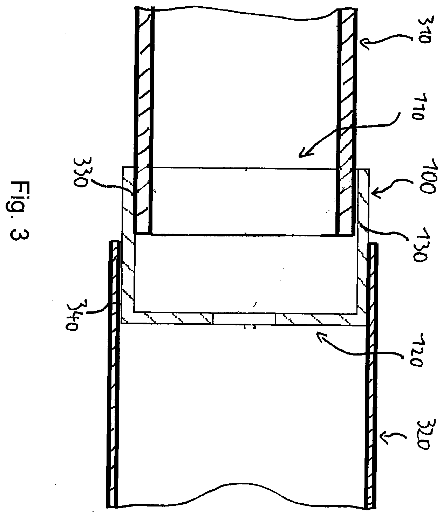

Fig. 3 zeigt eine schematische Ansicht des Querschnitts des Steriladapters 100 aus Fig. 1 mit angeschlossenem sterilisierbaren Endoskop 310 und Kamerakopf 320.

Das Endoskop 310 wird an dem distalen Ende 110 über eine erste Steckverbindung 330 in den Steriladapter 100 gesteckt. Im dargestellten eingesteckten Zustand umgibt der Tubus 130 einen Teil des Endoskops 310.

Der Kamerakopf 320 wird an dem proximalen Ende 120 des Steriladapters 100 über eine zweite Steckverbindung 340 auf den Steriladapter 100 gesteckt, so dass ein Teil des Kamerakopfes 320 den Tubus 130 umgibt. Im dargestellten Betriebsfall des Steriladapters 100 mit angeschlossenem Endoskop 310 und Kamerakopf 320 ist ein von außen zugänglicher und den Steriladapter 100 umgebender Bereich des Kamerakopfes 320 steril, während ein innenliegender und durch den Steriladapter geschützter Bereich des Kamerakopfes 320 unsteril ist.

In einem nicht dargestellten Ausführungsbeispiel ist an dem Tubus im Bereich des Längsabschnitts ein beweglicher Verschluss angeordnet, der ausgebildet ist, eine offene Verschlussstellung anzunehmen, wenn der Steriladapter an das Endoskop angeschlossen ist, und eine geschlossene Verschlussstellung anzunehmen, wenn der Steriladapter nicht an das Endoskop angeschlossen ist. Hierbei behindert die geschlossene Verschlussstellung eine freie Lichtausbreitung zwischen dem Endoskop und dem Kamerakopf und die offene Verschlussstellung erlaubt die freie Lichtausbreitung.

In einem weiteren nicht dargestellten Ausführungsbeispiel ist die erste und/oder die zweite Steckverbindung so ausgebildet, dass ein Teil des Endoskops und/oder ein Teil des Kamerakopfs im angeschlossenen Zustand den Tubus umgibt, wobei weiterhin ein Steg auf der Mantelfläche des Tubus in einer Ebene senkrecht zur Längsachse ausgebildet ist, so dass der Steg eine Bewegung des Endoskops und/oder des Kamerakopfes entlang des Tubus in Richtung der Längsachse behindert und dadurch eine jeweilige Verbindungstiefe der ersten und/oder zweiten Steckverbindung begrenzt. Der Steg kann insbesondere verhindern, dass sich Endoskop und Kamerakopf in einem jeweils angeschlossenen Zustand auf der Mantelfläche des Steriladapters berühren.

### Bezugszeichenliste:

- 100: Steriladapter
- 110: distales Ende
- 120: proximales Ende
- 130: Tubus
- 135: Mantelfläche
- 136: Innenkontur
- 138: eingeschlossener Innenraum
- 140: Längsabschnitt
- 150: Längsachse
- 160: Öffnung
- 165: Mittelpunkt
- 170: Öffnungswinkel
- 310: sterilisierbares Endoskop
- 320: Kamerakopf
- 330: erste Steckverbindung
- 340: zweite Steckverbindung
- D1: Innendurchmesser der Öffnung
- D2: Durchmesser der Innenkontur
- S1: Stärke des Tubus
- S2: Stärke der Lochblende

## Patentansprüche

1. Steriladapter (100) zur Anordnung zwischen einem sterilisierbaren Endoskop (310) und einem Kamerakopf (320), wobei der Steriladapter ein an das Endoskop (310) anschließbares distales Ende (110) und ein an den Kamerakopf (320) anschließbares proximales Ende (120) umfasst, wobei der Steriladapter (100) einen Tubus (130) mit einer Längsachse (150) aufweist, wobei ein Längsabschnitt (140) des Tubus (130) einen in einer Querschnittebene senkrecht zur Längsachse (150) verringerten Innendurchmesser aufweist, der eine zentrale Öffnung (160) bildet, die so bemessen ist, dass eine freie Lichtausbreitung zwischen dem Endoskop (310) und dem Kamerakopf (320) entlang der Längsachse (150) möglich aber ein Durchgreifen durch einen von dem Tubus (130) eingeschlossenen Innenraum (138) des Steriladapters (100) erschwert ist, **dadurch gekennzeichnet, dass** die zentrale Öffnung (160) des Längsabschnitts (140) einen Innendurchmesser von höchstens 10mm aufweist, und wobei der Tubus (130) an seinem distalen Ende (110) einen Innendurchmesser von weniger als 30mm hat und ein Abstand der zentralen Öffnung (160) von dem distalen Ende (110) des Tubus (130) größer ist als die Hälfte eines Innendurchmessers des Tubus (130) an dessen distalem Ende (110), wobei der Tubus (130) sich vom distalen Ende (110) zum proximalen Ende (120) erstreckt und der Längsabschnitt (140) des Tubus (130) das proximale Ende (120) bildet.

2. Steriladapter (100) gemäß Anspruch 1, bei dem die zentrale Öffnung (160) des Längsabschnitts (140) einen Innendurchmesser zwischen 1mm und 10mm, insbesondere zwischen 4mm und 8mm aufweist.

3. Steriladapter (100) gemäß Anspruch 1 oder 2, bei dem der Tubus (130) in Richtung der Längsachse (150) eine Ausdehnung von mindestens 10mm und vorzugsweise mehr als 30mm aufweist.

4. Steriladapter (100) gemäß mindestens einem der vorherigen Ansprüche, bei dem der Längsabschnitt (140) mit dem in einer Querschnittebene senkrecht zur Längsachse (150) verringertem Innendurchmesser in Richtung der Längsachse (150) eine Breite zwischen 0,5mm und 2mm aufweist.

5. Steriladapter (100) gemäß mindestens einem der vorherigen Ansprüche, bei dem der Längsabschnitt (140) mit verringertem Innendurchmesser als Lochblende ausgebildet ist.

6. Steriladapter (100) gemäß mindestens einem der vorherigen Ansprüche, der weitgehend rotationssymmetrisch zur Längsachse (150) ausgebildet ist.

7. Steriladapter (100) gemäß mindestens einem der vorherigen Ansprüche, bei dem der Steriladapter (100) monolithisch ausgebildet ist.

8. Steriladapter (100) gemäß mindestens einem der vorherigen Ansprüche, bei dem der Tubus (130) in Richtung der Längsachse (150) eine Ausdehnung hat, die größer als das 0,8-fache des maximalen Innendurchmessers des Tubus (130) in einer Richtung senkrecht zur Längsachse (150) ist.

9. Steriladapter (100) gemäß mindestens einem der vorherigen Ansprüche, bei dem die Öffnung (160) an dem proximalen Ende (120) des Steriladapters (100) ausgebildet ist.

10. Steriladapter (100) gemäß mindestens einem der vorherigen Ansprüche, bei dem an dem Tubus (130) im Längsabschnitt (140) ein beweglicher Verschluss der Öffnung (160) angeordnet ist, der ausgebildet ist eine offene Verschlussstellung anzunehmen, wenn der Steriladapter (100) mechanisch mit dem Endoskop (310) verbunden ist, und eine geschlossene Verschlussstellung anzunehmen, wenn der Steriladapter (100) nicht mechanisch mit dem Endoskop (310) verbunden ist, wobei die geschlossene Verschlussstellung die freie Lichtausbreitung zwischen dem Endoskop (310) und dem Kamerakopf (320) behindert und die offene Verschlussstellung die freie Lichtausbreitung erlaubt.

11. Steriladapter (100) gemäß mindestens einem der vorherigen Ansprüche, wobei der Steriladapter (100) und das Endoskop (310) und/oder der Kamerakopf (320) über eine durch den Tubus (130) jeweils gebildete Steckverbindung aneinander anschließbar sind.

12. Steriladapter (100) gemäß mindestens einem der vorherigen Ansprüche, wobei der Steriladapter (100) und das Endoskop (310) und/oder der Kamerakopf (320) über eine durch den Tubus (130) jeweils gebildete Schraubverbindung aneinander anschließbar sind.

## Claims

1. A sterile adapter (100) for arrangement between a sterilizable endoscope (310) and a camera head (320), the sterile adapter comprising a distal end (110) which is attachable to the endoscope (310), and a proximal end (120) which is attachable to the camera head (320), wherein the sterile adapter (100) has a tube (130) with a longitudinal axis (150), wherein a longitudinal portion (140) of the tube (130) has an internal diameter which is reduced in a cross-sectional plane perpendicular to the longitudinal axis (150) and which forms a central opening (160) which is dimensioned such that, while a free propagation of light is possible between the endoscope (310) and the camera head (320) along the longitudinal axis (150), it is nonetheless made difficult to reach through an interior (138) of the sterile adapter (100) enclosed by the tube (130), **characterized in that** the central opening (160) of the longitudinal portion (140) has an internal diameter of at most 10 mm, and wherein the tube (130) has an internal diameter of less than 30 mm at its distal end (110), and a distance of the central opening (160) from the distal end (110) of the tube (130) is greater than half an internal diameter of the tube (130) at the distal end (110) thereof wherein the tube (130) extends from the distal end (110) to the proximal end (120) and the longitudinal portion (140) forms the proximal end (130) of the tube (130).

2. The sterile adapter (100) as claimed in claim 1, in which the central opening (160) of the longitudinal portion (140) has an internal diameter of between 1 mm and 10 mm, in particular of between 4 mm and 8 mm.

3. The sterile adapter (100) as claimed in claim 1 or 2, in which the tube (130) has, in the direction of the longitudinal axis (150), an extent of at least 10 mm and preferably of more than 30 mm.

4. The sterile adapter (100) as claimed in at least one of the preceding claims, in which the longitudinal portion (140) of reduced internal diameter in a cross-sectional plane perpendicular to the longitudinal axis (150) has a width of between 0.5 mm and 2 mm in the direction of the longitudinal axis (150).

5. The sterile adapter (100) as claimed in at least one of the preceding claims, in which the longitudinal portion (140) of reduced internal diameter is configured as a pinhole stop.

6. The sterile adapter (100) as claimed in at least one of the preceding claims, which sterile adapter (100) is largely rotationally symmetrical with respect to the longitudinal axis (150).

7. The sterile adapter (100) as claimed in at least one of the preceding claims, in which the sterile adapter (100) is of monolithic form.

8. The sterile adapter (100) as claimed in at least one of the preceding claims, in which the tube (130) has, in the direction of the longitudinal axis (150), an extent that is greater than 0.8 times the maximum internal diameter of the tube (130) in a direction perpendicular to the longitudinal axis (150).

9. The sterile adapter (100) as claimed in at least one of the preceding claims, in which the opening (160) is formed at the proximal end (120) of the sterile adapter (100).

10. The sterile adapter (100) as claimed in at least one of the preceding claims, in which a movable shutter of the opening (160) is arranged on the tube (130) in the longitudinal portion (140) and is designed to adopt an open shutter position when the sterile adapter (100) is mechanically connected to the endoscope (310), and to adopt a closed shutter position when the sterile adapter (100) is not mechanically connected to the endoscope (310), wherein the closed shutter position obstructs the free propagation of light between the endoscope (310) and the camera head (320), and the open shutter position permits the free propagation of light.

11. The sterile adapter (100) as claimed in at least one of the preceding claims, wherein the sterile adapter (100) and the endoscope (310) and/or the camera head (320) are attachable to each other via a plug connection formed in each case by the tube (130).

12. The sterile adapter (100) as claimed in at least one of the preceding claims, wherein the sterile adapter (100) and the endoscope (310) and/or the camera head (320) are attachable to each other via a screw connection formed in each case by the tube (130).

## Revendications

1. Adaptateur (100) stérile à monter entre un endoscope (310) stérilisable et une tête (320) d'appareil photographique, dans lequel l'adapteur (100) stérile comprend une extrémité (110) distale pouvant être raccordée à l'endoscope (310) et une extrémité (120) proximale pouvant être raccordée à la tête (320) de l'appareil photographique, dans lequel l'adaptateur (100) stérile a un tube (130) ayant un axe (150) longitudinal, dans lequel un tronçon (140) longitudinal du tube (130) a un diamètre intérieur, qui est diminué dans un plan de section transversale perpendiculaire à l'axe (150) longitudinal et qui forme une ouverture (160) centrale dimensionnée de manière à ce qu'une propagation libre de la lumière entre l'endoscope (310) et la tête (320) de l'appareil le long de l'axe (150) longitudinal soit possible, mais qu'une pénétration dans un espace (138) intérieur, enfermé par le tube (130) de l'adaptateur (100) stérile, soit rendue difficile,
**caractérisé en ce que** l'ouverture (160) centrale du tronçon (140) longitudinal a un diamètre intérieur de 10mm au plus et dans lequel le tube (130) a à son extrémité (110) distale un diamètre intérieur de moins de 30 mm et une distance de l'ouverture (160) centrale à l'extrémité (110) distale du tube est plus grande que la moitié d'un diamètre intérieur du tube à son extrémité (110) distale dans lequel le tube (130) s'étend de l'extrémité (110) distale à l'extrémité (130) proximale et le tronçon (140) longitudinal du tube (130) forme l'extrémité (120) proximale.

2. Adaptateur (100) stérile suivant la revendication 1, dans lequel l'ouverture (160) centrale du tronçon (140) longitudinal a un diamètre intérieur compris entre 1mm et 10mm, en étant notamment compris entre 4mm et 8mm.

3. Adaptateur (100) stérile suivant la quelconque revendication 1 ou 2, dans lequel le tube (130) a dans la direction de l'axe (150) longitudinal une étendue d'eau moins 10mm et de préférence de plus de 30 mm.

4. Adaptateur (100) stérile suivant au moins l'une quelconque des revendications précédentes, dans lequel le tronçon (140) longitudinal ayant le diamètre intérieur diminué dans un plan de section transversale perpendiculaire à l'axe (150) longitudinal a dans la direction de l'axe (150) longitudinal une largeur comprise entre 0,5mm et 2mm.

5. Adaptateur (100) stérile suivant au moins l'une quelconque des revendications précédentes, dans lequel le tronçon (140) longitudinal de diamètre intérieur diminué est constitué sous la forme d'un diaphragme perforé.

6. Adaptateur (100) stérile suivant au moins l'une quelconque des revendications précédentes, qui est constitué dans une grande mesure de révolution par rapport à l'axe (150) longitudinal.

7. Adaptateur (100) stérile suivant au moins l'une quelconque des revendications précédentes, dans lequel l'adaptateur (100) stérile est constitué de manière monolithique.

8. Adaptateur (100) stérile suivant au moins l'une quelconque des revendications précédentes, dans lequel le tube (130) a une étendue dans la direction de l'axe (150) longitudinal, qui est plus grande que 0,8 fois le diamètre intérieur maximum du tube dans une direction perpendiculaire à l'axe (150) longitudinal.

9. Adaptateur (100) stérile suivant au moins l'une quelconque des revendications précédentes, dans lequel l'ouverture (160) est constituée à l'extrémité (120) de l'adaptateur (100) stérile.

10. Adaptateur (100) stérile suivant au moins l'une quelconque des revendications précédentes, dans lequel sur le tube (130) est constitué dans la direction (140) longitudinale une fermeture mobile de l'ouverture (160), qui est constituée pour prendre une position de fermeture ouverte, lorsque l'adaptateur (100) stérile est relié mécaniquement à l'endoscope (310), et pour prendre une position de fermeture fermée, lorsque l'adaptateur n'est pas relié mécaniquement à l'endoscope (310), la position de fermeture fermé empêchant la propagation libre de la lumière entre l'endoscope (310) et la tête (320) de l'appareil photographique et la position de la fermeture ouverte permettant la propagation libre de la lumière.

11. Adaptateur (100) stérile sur au moins l'une quelconque des revendications précédentes, dans lequel l'adaptateur (100) stérile et l'endoscope (310) et/ou la tête (320) de l'appareil photographique peuvent être raccordé l'un à l'autre par une liaison à emmanchement formée respectivement par le tube (130).

12. Adaptateur (100) stérile sur au moins l'une quelconque des revendications précédentes, dans lequel l'adaptateur (100) stérile et l'endoscope (310) et/ou la tête (320) de l'appareil photographique peuvent être raccordés l'un à l'autre par un vissage formé respectivement par le tube (130).
